(19) Europäisches Patentamt
European Patent Office
Office européen des brevets

(11) **EP 3 717 890 B1**

(12) **EUROPEAN PATENT SPECIFICATION**

(45) Date of publication and mention
of the grant of the patent:
**28.02.2024 Bulletin 2024/09**

(21) Application number: **17933601.1**

(22) Date of filing: **01.12.2017**

(51) International Patent Classification (IPC):
**G01N 21/00** (2006.01)   **C07C 2/20** (2006.01)
**C07C 11/02** (2006.01)   **G05D 11/00** (2006.01)
**C07C 2/30** (2006.01)   **G01N 21/33** (2006.01)
**G05D 21/02** (2006.01)

(52) Cooperative Patent Classification (CPC):
(C-Sets available)
**C07C 2/30; G05D 21/02;** G01N 21/33     (Cont.)

(86) International application number:
**PCT/RU2017/000890**

(87) International publication number:
**WO 2019/108081 (06.06.2019 Gazette 2019/23)**

(54) **OLEFIN OLIGOMERIZATION METHOD USING ONLINE MEASUREMENT OF CHROMIUM CONCENTRATION IN A CATALYTIC SYSTEM**

VERFAHREN ZUR OLIGOMERISIERUNG VON OLEFINEN UNTER VERWENDUNG EINER ONLINE-MESSUNG DER CHROMKONZENTRATION IN EINEM KATALYTISCHEN SYSTEM

PROCÉDÉ D'OLIGOMÉRISATION D'OLÉFINES UTILISANT UNE MESURE EN LIGNE DE CONCENTRATION DE CHROME DANS UN SYSTÈME CATALYTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.10.2020 Bulletin 2020/41**

(73) Proprietor: **Public Joint Stock Company "Sibur Holding"**
**Tyumen region 626150 (RU)**

(72) Inventors:
• **LENEV, Denis Alekseevich**
**Khimki**
**Moskovskaya obl. 147407 (RU)**
• **DOMRACHEVA, Diana Sergeevna**
**Tomsk 634003 (RU)**

(74) Representative: **Peters, Andreas**
**Hannke Bittner & Partner**
**Patent- und Rechtsanwälte mbB**
**Prüfeninger Straße 1**
**93049 Regensburg (DE)**

(56) References cited:
**WO-A1-2016/105227     US-A1- 2007 043 181**

• **KALIDHASAN S ET AL: "Simple and selective extraction process for chromium (VI) in industrial wastewater", JOURNAL OF HAZARDOUS MATERIALS, ELSEVIER, AMSTERDAM, NL, vol. 170, no. 2-3, 30 October 2009 (2009-10-30), pages 1079-1085, XP026521239, ISSN: 0304-3894, DOI: 10.1016/J.JHAZMAT.2009.05.071 [retrieved on 2009-05-22]**
• **SKOBELEV IGOR. Y. et al.: "In Situ EPR Monitoring of Chromium Species Formed during Cr-Pyrrolyl Ethylene Trimerization Catalyst Formation", Organometallics, vol. 29 , pages 2943-2950, XP055617346,**
• **SOARES RICARDO et al.: "Simultaneous speciation of chromium by spectrophotometry and multicomponent analysis", Chemical Speciation and Bioavailability, vol. 21, no. 3, January 2009 (2009-01), pages 153-160, XP055617349,**

EP 3 717 890 B1

(52) Cooperative Patent Classification (CPC): (Cont.)

C-Sets
**C07C 2/30, C07C 11/107**

## Description

## Technical field of the invention

The invention relates to the field of olefin oligomerization for producing linear $\alpha$-olefins, in particular hexene-1, used for production of low, average and high density linear polyethylene, poly-$\alpha$-olefins for drag reducing additives etc. In particular, the invention relates to a method of controlling and regulating the process of dilution and dosing of the catalytic system to the olefin oligomerization reactor by online measurement of concentration of the olefin oligomerization catalytic system.

## Background of the invention

Carrying out monitoring and control of dosing of starting reactants into an oligomerization reactor is one of important factors for effectively controlling the olefin oligomerization process. In particular, control of the concentration of a catalytic system fed to the oligomerization reactor is required for providing efficiency and reliability of the manufacturing process. Thus, it is known from the art that the concentration of a catalytic system fed to the olefin oligomerization reactor directly affects activity, selectivity and productivity of the olefin oligomerization process (see, e.g. WO2016105227 application, Examples 1.A.1-1.A.2). Therefore, it is necessary to control catalytic system concentration after its dilution by determining the contents of chromium compounds prior to feeding the catalytic system to the olefin oligomerization reactor.

One variant of determination of concentration for the active components of the catalytic system is sampling of the small amount of catalytic system with subsequent analytical testing in a remote laboratory. Wherein, the sampling process itself is difficult and dangerous because it is necessary to sample from the stream of organometallic compounds. Moreover, there is a risk of the fact that the sample would not be representative, i.e. that the sample would not reflect the correct content and concentration of the catalytic system, and removal of some amount of catalytic system from the process might result in alteration of its selectivity and productivity. In addition, sampling takes a prolonged time, due to which analytical data for the sample obtained with a delay only have a limited value for rapid and effective control of a technological process.

Therefore, a preferable method of controlling the concentration of catalytic system dosed to the olefin oligomerization reactor is the control of the concentration by online method, i.e. directly during the dosing of the catalytic system components to the olefin oligomerization reactor or after such dosing, or, when possible, immediately after dilution. Such online method would allow for rapidly obtaining the required measurements, as well as would increase safety of the manufacturing process.

Various methods for monitoring and controlling chemical technology processes are known using physical and chemical measurements of concentrations of various compounds. Thus, in order to measure the concentrations of compounds in a stream various methods not disturbing the occurring process are used. Such methods involve, in particular, spectroscopy, such as infrared spectroscopy (IR spectroscopy), ultraviolet spectroscopy (UV spectroscopy), Raman spectroscopy (combination scattering spectroscopy). Moreover, such methods may also involve the kinds of spectroscopy such as nuclear magnetic resonance (NMR), electronic paramagnetic resonance (EPR), which are, however, more difficult and require higher costs.

The patents US7315369, US7396970, US7505129, US9040605 provide a method for monitoring and controlling concentrations of reactants and products of oligomerization or polymerization reaction of olefins and other chemical compounds using Raman spectroscopy method. The equipment for olefin oligomerization or polymerization comprises: at least one probe for Raman spectroscopy located before, after or in the oligomerization or polymerization reactor, equipment for Raman spectroscopy located outside the oligomerization or polymerization reactor and connected to the at least one probe for Raman spectroscopy. The method comprises: a) contacting a monomer and catalytic system in the reaction zone under desired conditions with b) producing an oligomer or a polymer, c) carrying out the first measurement of residual monomer concentration using the Raman spectroscopy equipment, and d) determining properties of the obtained product and correcting, if necessary, at least one condition of the oligomerization or polymerization process in response to the first measurement. This method allows for effectively *in situ* controlling the concentration of the starting monomer and the obtained target oligomer or polymer and thus allows for providing the required conditions for carrying out the olefin oligomerization or polymerization processes; however, there is no data in the said patents about a possibility of using such method for determination of catalytic system concentration.

A method is known for monitoring chrome particles formed in catalytic system used in the ethylene trimerization process using EPR spectroscopy method (Igor Y. Skobelev, Valentina N. Panchenko, Oleg Y. Lyakin, Konstantin P. Bryliakov, Vladimir A. Zakharov, Evgenii P. Talsi. In situ EPR monitoring of chromium species formed during Cr-pyrrolyl ethylene trimerization catalyst formation. Organometallics, 2010, 29 (13), pp. 2943-2950). Said catalytic system comprises a chromium source, in particular chromium (III) acetyl acetonate, pyrrole, triethyl aluminium and diethyl aluminium chloride in cyclohexane. The method allows for determining the dependency of activity of the ethylene trimerization process from concentration of chromium particles. However, the experimental data demonstrate that only 20% of the total amount of chromium present in the system can be determined using said method. Therefore, said method is ineffective for determination of concentration and total amount of chromium present in the system because most part of the chromium is present in diamagnetic state and therefore cannot be determined using EPR spectroscopy method.

Moreover, a method is known for determining the absorption of UV-visible light by solutions of organometallic chromium complexes in particular those being individual diazachromocene compounds (Markus Kreye, Constantin G. Daniliuc, Matthias Freytag, Peter G. Jones, Marc D. Walter. Coordination chemistry of sterically encumbered pyrrolyl ligands to chromium (II): mono(pyrrolyl)-chromium and diazachromocene formation. Dalton Trans, 2014, 43, pp. 9052-9060). Wherein, UV spectroscopy is used in order to study the structure of chromium organometallic complexes. However, in said document, there is no data about a possibility of using the results of measurements obtained by the UV spectroscopy method for determination of the chromium amount of in the said solutions. Also, measurement of absorption of UV-visible radiation of organometallic complexes that are individual diazachromocene compounds is practiced in said method, while a possibility of analyzing solutions of more complex contents comprising a mixture of various chromium complexes is not mentioned.

A problem related to the chromium-pyrrole catalytic system used in the olefin oligomerization process is the fact that the catalytic system contains 30 to 50-fold excess of organometallic compounds that can form various complexes with pyrrole ligands, and the chromium complex itself is believed to be a mixture of undefined composition. Therefore, it is possible that upon alteration in concentration of catalytic system chemical composition thereof alters as well, thus significantly hindering a possibility of determining chromium concentration in such a system.

Therefore, online methods for measuring concentrations of various substances including those during the olefin oligomerization processes as known from the art are insufficiently effective.

In this connection, one promising field is a development of an olefin oligomerization process wherein an online control of the concentration and amount of catalytic system after its dilution and prior to feeding the system to the olefin oligomerization reactor would be carried out.

Summary of the invention

Thus, the object of the present invention is to develop a method for online controlling the concentration and amount of catalytic system used in the olefin oligomerization prior to feeding thereof to the olefin oligomerization reactor, and also an olefin oligomerization method characterized by increased selectivity, productivity and decrease in the amount of by-products.

The technical result of the present invention consists in realizing a process of oligomerization of olefins wherein an online control of concentration of catalytic system fed to the oligomerization reactor is carried out, which, in turn, allows for providing high selectivity and productivity of the olefin oligomerization process.

**Said technical problem is solved and achievement of the desired technical result is provided due to** using, in the olefin oligomerization process, a method of online control of chromium concentration in the catalytic system after dilution thereof, by measuring the wavelength in the range of 400 nm to 300 nm and recording the absorption in the UV cell disposed in the dilution vessel or pipeline after at least 60 minutes starting from preparation of the catalytic system solution.

Therefore, in a first aspect the present invention relates to an online method for determining chromium concentration in olefin oligomerization catalytic system comprising a chromium source, a nitrogen-containing ligand and alkyl aluminium, the method comprising:

- diluting a catalytic system with hydrocarbon solvent to a required total chromium concentration ranging from 0.5 to 10 mg/l; and
- determining chromium concentration in the diluted catalytic system by measuring optical density of the obtained diluted solution in the wavelength range of 400 to 300 nm after at least 60 min starting from producing the catalytic system.

The method according to the first aspect of the present invention is defined in claim 1.

In a second aspect the invention relates to a method of controlling chromium concentration in olefin oligomerization catalytic system comprising a chromium source, a nitrogen-containing ligand and alkyl aluminium, the method comprising:

- diluting a catalytic system comprising a chromium source, a nitrogen-containing ligand and alkyl aluminium with hydrocarbon solvent to a required concentration ranging from 0.5 to 10 mg/l; and
- online determining chromium concentration in the diluted catalytic system by a method described above; and
- correcting, if necessary, the amount of solvent fed for dilution of the catalytic system and/or the amount of concentrated catalytic system in response to the performed measurement.

The method according to the second aspect of the invention is defined in claim 15.

In a third aspect the present invention relates to a process of olefin oligomerization that comprises the steps of:

   a) producing a catalytic system by mixing a chromium source, a nitrogen-containing ligand and an alkyl aluminium,
   b) diluting the catalytic system obtained in step a) with hydrocarbon solvent to a required concentration;

c) feeding a diluted catalytic system, a feedstock comprising $\alpha$-olefin and optionally a zinc compound to an oligomerization reactor;

d) reacting the feedstock comprising $\alpha$-olefin under oligomerization conditions in the presence of a catalytic system comprising the chromium source, the nitrogen-containing ligand, alkyl aluminium and optionally zinc compounds,

the process being characterized in that chromium concentration in a diluted catalytic system is controlled by the method as described above, prior to feeding the diluted catalytic system to the oligomerization reactor (step c).

The method according to the third aspect of the invention is defined in claim 16.

A fourth aspect of the invention concerns a device for controlling the chromium concentration in the olefin oligomerization system comprising a chromium source, a nitrogen-containing ligand and alkyl aluminium comprising: a catalytic system preparation unit connected downstream via a regulating flow meter with a mixing unit for mixing the catalytic system and a solvent; a reservoir with a hydrocarbon solvent for diluting the catalytic system, connected downstream via a regulating flow meter with a mixing unit for mixing the catalytic system and the solvent; UV analyzer designed for measuring the optical density of the catalytic system diluted solution in a wavelength range of 400 to 300 nm after the diluted catalytic system exits the mixing unit; and a feedback unit for carrying out correction of volumetric flow rates of the solvent fed from the solvent reservoir to the mixing unit (according to positive feedback mechanism) and/or the catalyst solution fed from the catalytic system preparation unit to the mixing unit (according to negative feedback mechanism) in response to measurement made by UV analyzer. The device of the present invention is defined in claim 34. Moreover, the invention also relates to use of such a device in the aforesaid method for olefin oligomerization.

The present invention allows for significantly simplifying the control of the catalytic system concentration after dilution thereof and also of the amount of diluted catalytic system fed to the olefin oligomerization reactor, thereby allowing, in turn, for providing high selectivity and productivity of the olefin oligomerization process.

## Description of figures

In order to explain the technical solutions and further disclose the essence of the present invention Figs 1 to 4 are represented.

Fig. 1 represents a flow chart demonstrating a sequence of operations in the methods of the present invention.

Fig. 2 represents a flow chart illustrating a device of the present invention.

Fig. 3 represents kinetics of formation of the olefin oligomerization catalytic system.

Fig. 4 represents calibration plots of the optical density of the catalytic system solution when absorbing light at wavelengths of 343 nm and 300 nm vs. chromium concentration.

## Detailed description of the invention

A detailed description of various aspects of realization of the present invention is further given.

In accordance with the present invention, a process of olefin oligomerization comprises the following steps:

a) producing a catalytic system by mixing a chromium source, a nitrogen-containing ligand and an alkyl aluminium,

b) diluting the catalytic system obtained in step (a) with hydrocarbon solvent to a required concentration;

c) feeding diluted catalytic system, a feedstock comprising $\alpha$-olefin and optionally a zinc compound to an oligomerization reactor;

d) reacting the feedstock comprising $\alpha$-olefin under oligomerization conditions in the presence of a catalytic system comprising the chromium source, the nitrogen-containing ligand, alkyl aluminium and optionally zinc compounds.

Wherein, the said method is characterized by that determination of chromium concentration in the diluted catalytic system by means of measuring optical density of the obtained diluted solution in the wavelength range of 400 to 300 nm at least 60 min after producing the catalytic system is performed and, if necessary, correction of the and/or the amount of concentrated catalytic system in response to the performed measurement is carried out prior to feeding the diluted catalytic system to the oligomerization reactor (step c).

In accordance with the present invention the olefin oligomerization is carried out in the presence of a catalytic system comprising chromium source, nitrogen-containing ligand and alkyl aluminium.

In order to realize the process of olefin oligomerization provided by the present invention, the catalytic system for olefin oligomerization comprising the chromium source, the nitrogen-containing ligand and alkyl aluminium is diluted with hydrocarbon solvent prior to feeding to the oligomerization reactor.

The catalytic system is usually diluted to chromium concentration of 0.5 to 10 mg/l, preferably 2 to 5 mg/l. In case the chromium concentration would be above 10 mg/l it could lead to overheating of reaction mass due to impossibility of effective heat withdrawal and, as a consequence, to a selectivity decrease with respect to the target α-olefin. In case the chromium concentration would be below 0.5 mg/l it could lead to reduction in activity of the catalytic system, overcooling of the reaction mass, which, in its turn, would lead to decrease in the amount of the obtained α-olefin.

Therefore, even relatively small alteration in chromium concentration in the catalytic system may negatively affect the olefin oligomerization process. In this connection, the concentration and amount of chromium in the catalytic system after its dilution should be controlled prior to feeding the catalytic system to the olefin oligomerization reactor.

However, this process is hindered by the following factors:

> a) high labour intensity and harmfulness of sampling from the stream of organometallic compounds;
> b) duration of the process of sampling and performing analysis in a remote laboratory;
> c) complexity and uncertainty of contents of the catalytic system, namely of the chromium complexes formed during production of the catalytic system.

The present inventors have found that it is possible to determine chromium concentration in the catalytic system simply and effectively via online measurement of optical density of the catalytic system solution followed by analysis of the obtained measurement result.

The method proposed according to the present invention for determining chromium concentration in the catalytic system comprising chromium source, nitrogen-containing ligand and alkyl aluminium comprises:

- diluting of a catalytic system with hydrocarbon solvent to a required concentration; and
- determining chromium concentration in the diluted catalytic system by measuring optical density of the obtained diluted solution.

In turn, a method of controlling chromium concentration in the catalytic system according to the invention comprises:

- diluting a catalytic system comprising chromium source, nitrogen-containing ligand and alkyl aluminium with hydrocarbon solvent to a required concentration;
- determining chromium concentration in the diluted catalytic system by means of the aforesaid method of the present invention; and
- correcting, if necessary, the amount of solvent fed for dilution of the catalytic system and/or the amount of concentrated catalytic system in response to the performed measurement.

Aliphatic or cycloaliphatic hydrocarbons comprising 6 to 16 carbon atoms are used as hydrocarbon solvent for diluting the catalytic system. The hydrocarbon solvent may also comprise unsaturated hydrocarbons such as olefins or aromatic compounds. Suitable hydrocarbon solvents or solvent components are heptane, cyclohexane, decane, undecane, iso-decane fraction, hexene-1, Isopar™ (ExxonMobil) or mixtures thereof. It is especially preferable to use dilution by saturated hydrocarbon solvents or mixtures thereof because the presence of unsaturated or aromatic hydrocarbons in the reaction mixture during carrying out the oligomerization process may reduce activity of the catalytic system and increase the amount of by-products such as polymers, as it is known from the art (RU2104088 patent).

Determination of chromium concentration in a catalytic system after dilution thereof is carried out by measuring optical density of the catalytic system diluted solution in a closed-type cell, through which a stream of diluted catalytic system fed from the catalytic system and solvent mixing unit is continuously passing to the olefin oligomerization reactor, at the wavelength in a range of 400 to 300 nm in inert, air oxygen-free atmosphere. It is preferable to carry out the measurements at the wavelengths of 300 or 343 nm, most preferably 343 nm.

Measurement of the optical density in the UV cell for the diluted catalytic system solution is carried out at least 60 minutes after preparation of the diluted catalytic system solution. Measurement earlier than after 60 minutes can lead to incorrect determination of chromium concentration in the catalytic system due to uncompleted process of catalytic complex formation, which in its turn may lead to incorrect measurement data and also to disturbances during technological process due to use of an incompletely formed catalytic complex.

Controlling of the dilution degree of the catalytic system is carried out by altering the flow of the concentrated catalyst and solvent flow in accordance with the deviation of the observed value of catalytic system concentration from the required value of catalytic system concentration. In case of a higher value of the observed catalytic system concentration the solvent flow rate is increased (positive feedback) and/or the concentrated catalytic system flow rate is decreased (negative feedback), and, *vice versa,* in case of a lower value of the observed catalytic system concentration the solvent flow rate is decreased (positive feedback) and/or the concentrated catalytic system flow rate is increased (negative feedback).

The catalytic system diluted to the required concentration is then fed to the oligomerization reactor.

As it has been recited above, the catalytic system of the present invention comprises a chromium source, a nitrogen-containing ligand and an alkyl aluminium compound.

Wherein, as a chromium source comprised in the catalytic system, organic and/or inorganic chromium compounds may be used. Chromium oxidation state in the compounds may vary and can be 0, +1, +2, +3, +4, +5 or +6. Generally, the chromium source is a compound of general formula $CrX_n$ wherein X may be identical or different organic or inorganic substituents and n is an integer from 1 to 6. Organic X substituents may have 1 to 20 carbon atoms and may be an alkyl group, alkoxy group, carboxy group, acetyl acetonate, amino group, amido group etc. Suitable inorganic X substituents are chromium halides, chromium sulfates, chromium oxides etc. Nonlimiting examples of the chromium sources are: chromium (III) chloride, chromium (III) acetate, chromium (III) 2-ethylhexanoate, chromium (III) acetyl acetonate, chromium (III) pyrrolide, chromium (II) acetate, chromium (VI) dioxide dichloride ($CrO_2Cl_2$) etc.

The nitrogen-containing ligand comprised in the catalytic system is an organic compound comprising a pyrrole ring moiety, i.e. a five-membered aromatic ring having one nitrogen atom. Suitable nitrogen-containing ligands are, but not limited to: pyrrole, 2,5-dimethylpyrrole, lithium pyrrolide ($C_4H_4NLi$), 2-ethylpyrrole, 2-allylpyrrole, indole, 2-methylindole, 4,5,6,7-tetrahydroindole. It is most preferable to use pyrrole or 2,5-dimethylpyrrole.

The alkyl aluminium comprised in the catalytic system may be an alkyl aluminium compound, as well as halogenated alkyl aluminium compound, alkoxy alkyl aluminium compound and mixtures thereof. For increasing selectivity, use of said compounds that have been not in contact with water (not hydrolyzed) is preferable, said compounds being represented by general formulae $AlR_3$, $AlR_2X$, $AlRX_2$, $AlR_2OR$, $AlRXOR$ and/or $Al_2R_3X_3$, wherein R is an alkyl group, X is halogen atom. Suitable alkyl aluminium compounds are, but not limited to: triethyl aluminium, diethyl aluminium, tripropyl aluminium, triisobutyl aluminium, diethyl aluminium ethoxide and/or ethyl aluminium sesquichloride or mixtures thereof. It is most preferable to use triethyl aluminium or a mixture of triethyl aluminium and diethyl aluminium chloride.

According to the present invention, the said catalytic system may be obtained by mixing the chromium source and the nitrogen-containing ligand followed by mixing thereof with the alkyl aluminium. Wherein, it is preferable to activate the alkyl aluminium additionally prior to mixing by means of UHF radiation (microwave radiation).

Within the context of the present application the terms "UHF radiation/irradiation" and "microwave radiation/irradiation" are used interchangeably.

Mixing of the components of the catalytic system can be performed via any method known in the art. Mixing of the components of the catalytic system is carried out for 1 minute to 30 minutes, preferably no less than 2 minutes, more preferable no less than 4 minutes, no less than 8 minutes, no less than 15 minutes, most preferably no less than 25 minutes.

Alternatively, according to the present invention, the said catalytic system may be obtained by mixing the chromium source, nitrogen-containing ligand and alkyl aluminium, with the alkyl aluminium preferably having been subjected to activation by UHF-irradiation and being gradually fed to mixing with other components of the catalytic system directly from the reservoir subjected to UHF-radiation, so that the mixing time may be any suitable time, without the irradiated component losing special properties acquired under the effect of UHF-radiation.

The components of the catalytic system may be mixed in any order. Preferably, alkyl aluminium is added to a mixture of chromium source and nitrogen-containing ligand. Mixing of components is carried out in any suitable device known in the art, e.g. in a bubble-agitated apparatus, apparatus with stirrer, static mixer, in the presence of hydrocarbon solvent.

Suitable hydrocarbon solvents for mixing the components of the catalytic system are, but not limited to: hexene-1, benzene, toluene, ethyl benzene, xylene or mixtures thereof. It is preferable to use aromatic hydrocarbons as the solvents to promote increase of stability of the catalytic system and production of highly active and selective catalytic system. Preferably, the aromatic hydrocarbon solvent is selected from a group consisting of toluene, ethyl benzene or mixtures thereof. The most preferable aromatic hydrocarbon is ethyl benzene.

After the mixing and catalytic system production step has been carried out, it is possible to remove the hydrocarbon solvent from the mixture. It is known from the art (RU2104088 patent) that the presence of unsaturated or aromatic hydrocarbon in a reaction mixture during the oligomerization process may reduce the activity of catalytic system and increase the amount of by-products such as polymers. Solvent removal may be performed by any known method, e.g. creating a vacuum (evacuation). However, it should be noted that in case of carrying out the olefin oligomerization process at elevated temperatures the presence of unsaturated hydrocarbon solvent, such as e.g. ethyl benzene, may be even preferable because the said solvent increases stability of the catalytic system.

UHF irradiation of alkyl aluminium may be carried out both with respect to alkyl aluminium in a form of compound *per se,* predominantly in liquid state, and with respect to a solution of the alkyl aluminium compound in a hydrocarbon solvent, e.g. in hexane, cyclohexane, C10-C12 hydrocarbon fractions.

During irradiation, it is necessary for the components of the catalytic system to be activated to be present in a reservoir transparent to UHF radiation, e.g. in a reservoir made out of glass, PTFE, polypropylene.

The UHF radiation used may have a frequency in a range of 0.2 to 20 GHz. It is most preferable to use UHF radiation at a frequency of 2.45 GHz that does not generate radio interference and is widely used in household and industrial UHF radiation sources.

The nominal power of the UHF radiation is 1 W to 5000 W per 1 g of the alkyl aluminium used based on elemental aluminium.

In order to achieve the best results it is preferable for the irradiation time to be from 20 seconds to 20 minutes, preferably 15 minutes. Irradiation over 20 minutes usually does not give additional advantages for the properties of the obtained catalytic system. Irradiation below 20 seconds may be insufficient for substantial alteration of the properties of components to be activated, which, in turn, would lead to insufficient increase in activity and/or selectivity of the obtained catalytic system.

Mixing of the alkyl aluminium activated using UHF radiation (microwave radiation) with chromium source and nitrogen-containing ligand is carried out no more than 3 minutes after the irradiation stops, preferably no more than 1 minute after the irradiation stops.

In case the time period between mixing of the irradiated alkyl aluminium with chromium source and nitrogen-containing ligand is 3 minutes or more, properties of the obtained catalytic system deteriorate compared to system wherein the said period is less than 1 minute.

The component ratios of the catalytic system may vary. The aluminium:chromium molar ratio may be 5:1 to 500:1, preferably 10:1 to 100:1, most preferably 20:1 to 50:1. The ligand:chromium molar ratio may vary from 2:1 to 50:1, preferably 2.5:1 to 5:1.

The olefin oligomerization process is realized by reacting the hydrocarbon solvent diluted catalytic system described above with a feedstock, as which $\alpha$-olefins are used, and optionally with zinc compound.

The zinc compound may be used both as an individual compound and as a mixture with other compounds, e.g. in a form of hydrocarbon solution.

The zinc compound or mixture of such compounds is directly added to the oligomerization reactor. The zinc compound is used as additional activator of catalytic center, in particular, chromium.

The zinc compound may be metallic zinc, zinc-copper couple, activated zinc, alkyl zinc compounds, in particular, dimethyl, diethyl, and dibutyl zinc, aryl zinc compounds, such as diphenyl and ditolyl zinc, zinc amides, in particular zinc pyrrolides and zinc-porphyrin complexes, zinc oxygenates (including formate, acetate, basic acetate, 2-ethylhexanoate and other zinc carboxylates), zinc halides, in particular, anhydrous zinc chloride, or combinations thereof. It is preferable to use zinc compounds soluble in the solvents used in the oligomerization process.

The zinc:chromium molar ratio may vary and can be from 2:1 to 100:1, preferably 5:1 to 50:1.

$\alpha$-Olefins used in the olefin oligomerization method according to the present invention are preferably ethylene (ethene), propylene (propene) and butylene (butene).

The olefin oligomerization process is carried out in order to obtain higher olefins. Industrially important processes are processes for producing oligomeric $\alpha$-olefins from ethylene. $\alpha$-olefins are compounds with double carbon-carbon bond (C=C) at $\alpha$ position. Oligomeric $\alpha$-olefins obtained as a result of oligomerization process may comprise various C5-C40 olefins and mixtures thereof. E.g. oligomeric $\alpha$-olefins obtained as a result of ethylene oligomerization process can be hexene-1, octene-1, decene-1, dodecene-1, higher $\alpha$-olefins or mixtures thereof. It is preferable that the oligomerization process is an ethylene trimerization process to produce hexene-1.

The oligomerization process can be performed in any reactor known in the art. Suitable reactors are a continuous stirred tank reactor, batch reactor, ideal displacement reactor, tubular reactor. The reactor may be a gas-liquid reactor, e.g. an autoclave with stirrer, bubbling column (bubble reactor) with direct-flow or counter-flow of gas and liquid supply, as well as a bubbling gas lift reactor.

The pressure of the starting feedstock comprising olefin during the oligomerization process is usually 1 to 200 atm. In a preferable embodiment of the method wherein the oligomerization process is ethylene trimerization process producing hexene-1, ethylene pressure can be varied in a range of 1 to 200 atm, preferably 10 to 60 atm, most preferably 15 to 40 atm. It is preferable to increase ethylene pressure in order to increase oligomerization rate.

The temperature of the oligomerization process can usually vary in a range of from 0 inclusive to 160 °C inclusive, preferably from 40 inclusive to 130 °C inclusive. It is most preferable to maintain the temperature in the reactor in a range of from 80 inclusive to 120 °C inclusive. At this temperature, the polymeric by-product (in particular polyethylene) would not be precipitated from the solution and would be removed from the reactor as a solution, and the catalytic system would be most effective and selective. Carrying out the oligomerization process at higher temperatures (above 160 °C inclusive) can lead to deactivation of catalytic system.

In accordance with the proposed method, the reaction time may vary. The reaction time can be defined as feedstock and solvent residence time in the oligomerization reaction zone. When using continuous flow reactors, the reaction time can be defined as average feedstock and solvent residence time in the oligomerization reaction zone. The reaction time may be varied depending on the olefin used as feedstock, reaction temperature, reaction pressure and other process parameters. In some embodiments of the method the reaction time does not exceed 24 hours. The reaction time can be less than 12 hours, less than 6 hours, less than 3 hours, less than 2 hours, less than 1 h, less than 30 minutes, less than 15 minutes, less than 10 minutes. Most preferably, the reaction time is from 30 minutes to 90 minutes.

According to the proposed method the olefin and catalytic system may contact with hydrogen that is fed to the oligomerization reactor and used as diluent. Hydrogen can accelerate the oligomerization reaction and/or increase the activity of organometallic catalyst. Moreover, hydrogen may lead to decreasing the amount of the polymer formed as by-product and limit polymer precipitation on the equipment walls.

The olefin oligomerization process is usually carried out in the absence of water and oxygen.

The starting feedstock, the catalytic system diluted with the solvent and optionally zinc compound may be introduced into the oligomerization reactor in any order. It is preferable to introduce the components in the following sequence: a catalytic system diluted with the solvent, then optionally the zinc compound with subsequent dosing of the starting olefin.

According to the proposed method, the stream outgoing from the reactor can contain organometallic compounds, target products, by-products, a solvent, and also polymers that can be formed during oligomerization.

The olefin oligomers may comprise olefin isomers, and the weight ratio of the target oligomeric α-olefin to the corresponding isomers should be at least 99.5:0.5.

The stream outgoing from the reactor may be treated with an agent deactivating the catalytic system. Suitable deactivating agents known from the art are water, alcohols, amines, amino alcohols or mixtures thereof, as well as various sorbents such as silica gel, aluminium oxide, alumosilicates or mixtures thereof with water, alcohols, amines, amino alcohols. Methanol, ethanol, n-propanol, isopropanol, n-butanol, isobutanol, tert-butanol, 2-ethylhexanol, ethylene glycol, propylene glycol, triethylene glycol, polyethylene glycol or mixtures thereof can be used as the alcohols. Examples of suitable amines are ammonia, methyl amine, dimethyl amine, trimethyl amine, ethyl amine, diethyl amine, triethyl amine, tri-n-propyl amine, diisopropyl ethyl amine, tri-n-butyl amine, piperazine, pyridine, ethylene diamine, diethylene triamine or mixtures thereof. Examples of amino alcohols comprise ethanol amine, diethanol amine, triethanol amine, methyl diethanol amine, dodecyl diethanol amine, 1-amino-2-propanol or mixtures thereof. Preferably, alcohols or amino alcohols deposited on silica gel are used as the deactivating agents.

Additionally, the stream exiting the reactor may be cooled e.g. by passing it through a heat exchanger. Cooling of the stream exiting the reactor can comprise mixing the hot effluent stream with cooled effluent stream. Cooling of the effluent stream is performed to the temperature in the range of 20 inclusive to 100 °C inclusive, preferably to the temperature of less than 95 °C inclusive, less than 90 °C inclusive, less than 85 °C inclusive, less than 80 °C inclusive, less than 75 °C inclusive, less than 70 °C inclusive, less than 65 °C inclusive, less than 60 °C inclusive, less than 55 °C inclusive, less than 50 °C inclusive, less than 45 °C inclusive, less than 40 °C inclusive, less than 35 °C inclusive, less than 30 °C inclusive, less than 25 °C inclusive. Cooling of the effluent stream may be performed to ambient temperature, e.g. in the range of from 20 °C inclusive to 25 °C inclusive. The temperature to which the effluent stream is cooled, is selected in such a way that the precipitation of the polymer from the solvent is increased.

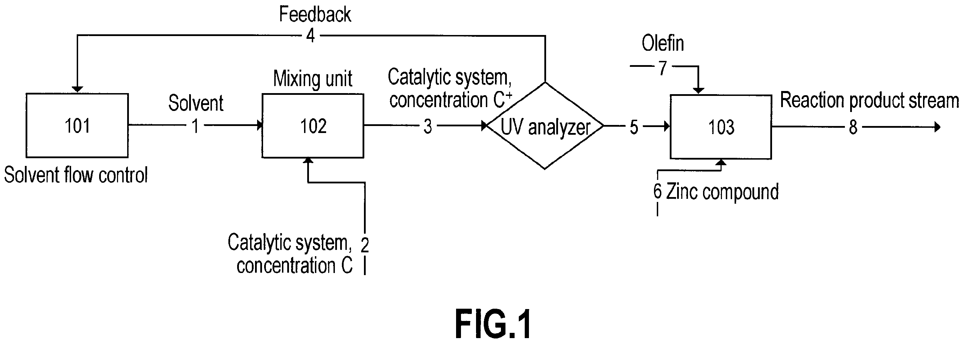

The technical essence of the proposed method according to the invention is outlined in more detail in **Fig. 1,** wherein **101** is a reservoir from which the solvent is dosed for diluting the catalytic system, **102** is a unit for mixing the catalytic system and the solvent, **UVanalyzer** is a device by means of which measurement of optical density of the catalytic system solution is carried out, **103** is the oligomerization reactor.

According to the outlined method, the solvent (1) from the reservoir 101 is fed to the unit 102 for mixing with catalytic system (2) furnishing the diluted catalytic system (3). Then, chromium concentration in the diluted catalytic system (3) is determined by measuring optical density of the obtained diluted solution using UV analyzer, and the amount of solvent (1) fed for dilution of the catalytic system (2) and/or the amount of diluted catalytic system (3) in response to the performed measurement is corrected when necessary - feedback (4). The obtained diluted catalytic system (5) is fed to the oligomerization reactor 103, to which feedstock (7) is also fed comprising α-olefin and optionally a zinc compound (6).

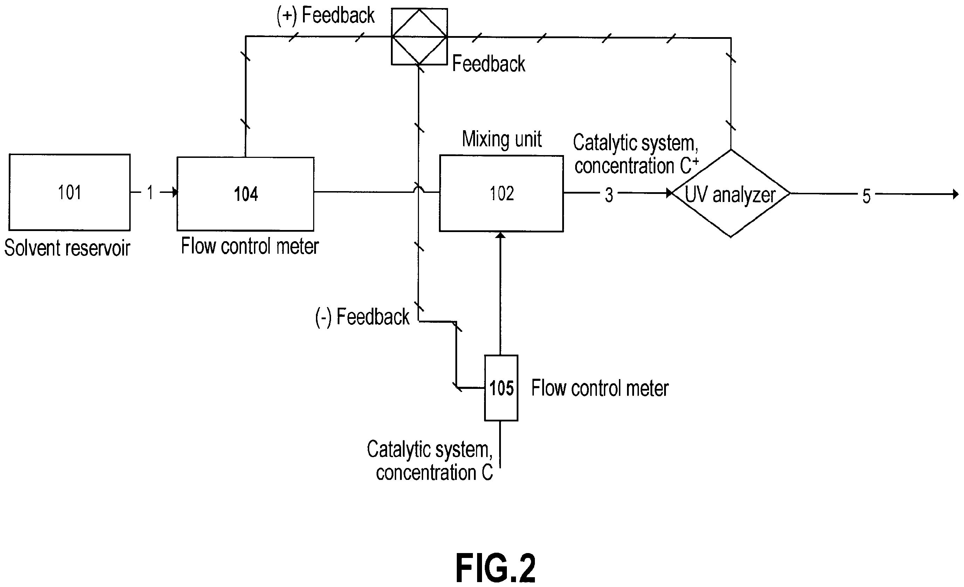

The technical essence of the proposed device is outlined in more detail in **Fig. 2** wherein **101** is a reservoir from which the solvent is dosed for diluting the catalytic system, **102** is the catalytic system and solvent mixing unit, **104** is flow meter controlling volumetric flow rate of the solvent, **105** is flow meter controlling volumetric flow rate of the catalytic system, **UV analyzer** is a device using which measurement of optical density of the catalytic system solution is carried out, **feedback unit** is a unit by means of which correction of the volumetric flow rate of solvent (according to positive feedback mechanism) and catalytic system solution (according to negative feedback mechanism) is carried out in response to measurement made by UV analyzer as described above.

The present invention allows for significantly simplifying control of the catalytic system concentration after its dilution, as well as of the amount of diluted catalytic system fed to the olefin oligomerization reactor. Maintaining the chromium concentration in the catalytic system at constant level allows for providing high selectivity and productivity of the olefin oligomerization process.

*This invention is described in more detail with a reference to the examples outlined below. The examples are intended for purposes of illustration of the present invention only and do not limit it in any way.*

## Examples of implementation of the invention

For carrying out the oligomerization reaction a catalytic system is used comprising 1) chromium (III) 2-ethylhexanoate as a chromium source, 2) 2,5-dimethylpyrrol as a nitrogen-containing ligand, 3) alkyl aluminium, namely triethyl aluminium (TEA) and diethyl aluminium chloride (DEAC), as an activator. Diethyl zinc compound is used as additional catalytic system activator. Upon exposure to said catalytic system the olefin oligomerization process occurs, in particular, ethylene trimerization, furnishing trimerization products.

**Carrying out the UV analysis**

Measurement of optical density of the catalytic system solution was carried out via UV spectroscopy method using the Cary Varian 50 spectrophotometer. The prepared catalytic system solution was transferred to the hermetically sealable cell in a nitrogen atmosphere, and scanning was conducted at room temperature and atmospheric pressure.

Scanning parameters:

Scanning rate: 600 nm/min
Scan step: 1 nm
Number of scanning cycles: 30 after each 2 min
Scanned range: 800 to 290 nm
Cell parameters: 10x10x50 mm, optical path length is 2 mm.

Measurements of the optical density of the catalytic system were carried out in relation to ethyl benzene absorption, i.e. the baseline correction was carried out using ethyl benzene.

Example 1. Kinetics of the catalytic system formation

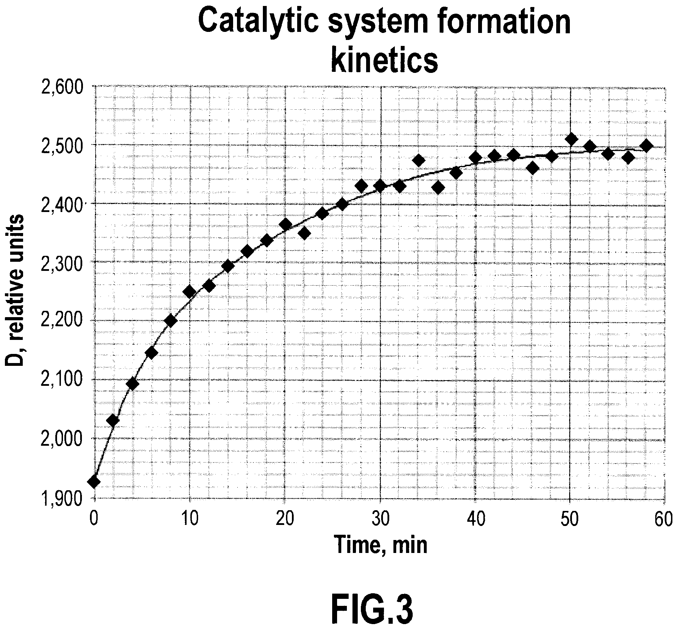

63% solution of chromium (III) 2-ethylhexanoate (0.29 g, 0.6 mmol), 2,5-diemthylpyrrol (0.11 g, 1.2 mmol) and 40 ml ethyl benzene were added into a glass flask under nitrogen atmosphere. To the obtained solution a mixture of 25% TEA solution in hexane (6.2 ml) and 15% DEAC solution in hexane (6.5 ml) were added that had been preliminarily irradiated in the MARS-6 microwave irradiator for 6 min. The obtained mixture was transferred after 3 min into a hermetically sealable quartz cell, and absorption spectra were recorded. Data showing the optical density increase are outlined in Table 1. Kinetics of catalytic system formation is outlined in Fig. 3.

Table 1 - data showing the increase of the UV absorption of the catalytic solution at the wavelength of 343 nm during formation of catalytic complex vs. time

| Analysis number | Time, min | Optical density |
|---|---|---|
| 1 | 0 | 1.928 |
| 2 | 2 | 2.032 |
| 3 | 4 | 2.092 |
| 4 | 6 | 2.144 |
| 5 | 8 | 2.200 |
| 6 | 10 | 2.249 |
| 7 | 12 | 2.259 |

(continued)

| Analysis number | Time, min | Optical density |
|---|---|---|
| 8 | 14 | 2.291 |
| 9 | 16 | 2.318 |
| 10 | 18 | 2.336 |
| 11 | 20 | 2.362 |
| 12 | 22 | 2.348 |
| 13 | 24 | 2.382 |
| 14 | 26 | 2.398 |
| 15 | 28 | 2.429 |
| 16 | 30 | 2.431 |
| 17 | 32 | 2.430 |
| 18 | 34 | 2.475 |
| 19 | 36 | 2.428 |
| 20 | 38 | 2.452 |
| 21 | 40 | 2.478 |
| 22 | 42 | 2.482 |
| 23 | 44 | 2.484 |
| 24 | 46 | 2.460 |
| 25 | 48 | 2.482 |
| 26 | 50 | 2.511 |
| 27 | 52 | 2.499 |
| 28 | 54 | 2.489 |
| 29 | 56 | 2.480 |
| 30 | 58 | 2.502 |

A conclusion can be made from the data outlined in Fig. 3 that absorption at the wavelength of 343 nm is stabilized after 60 min of withstanding the obtained catalytic system.

**Example 2. Dilution of the catalytic system and determination of the optical density of the solution**

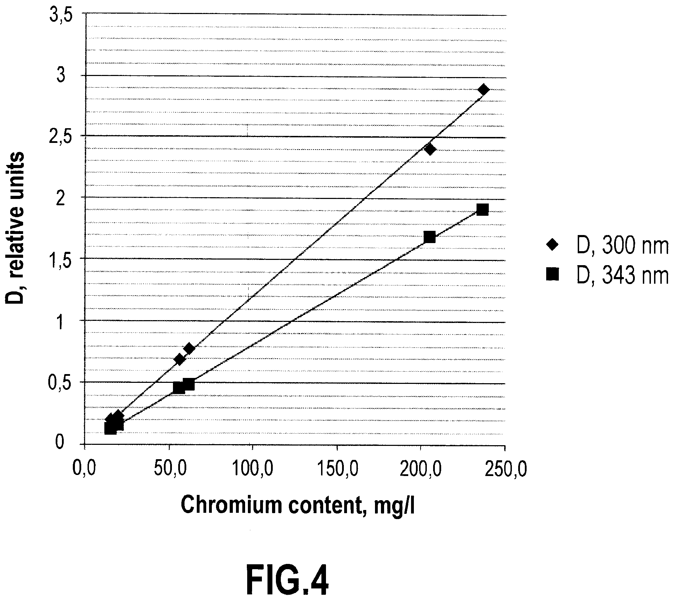

The catalytic system obtained according to technique described in Example 1 was diluted 2 to 100-fold with cyclohexane. Then UV spectra were recorded and optical absorption was measured in the range of 450 to 250 nm. Chromium concentration was then determined in the same solution using inductively coupled plasma mass spectrometry (ICP-MS) in the same solution. All operations were carried out in the nitrogen atmosphere. Basing on the obtained data a calibration curve was derived (Fig. 4).

Calculation of molar concentration of catalytic system is conducted using Bouguer-Lambert-Beer law

(1):

$$D = \varepsilon \cdot C \cdot l \ (1)$$

wherein D is optical density, $\lg(I_0/I)$; $\varepsilon$ is extinction coefficient (l/mol Cr*cm); c is solution concentration (mol/l); l is optical path length (cell thickness) (cm); $I_0$ is light intensity upon entering the substance; I is light intensity passed through the substance.

In order to calculate optical density of the catalytic system solution using calibration curve (Fig. 4), a dependence of the optical density of the catalytic system solution vs. chromium concentration was established as represented by formula (2) for the wavelength of 300 nm, formula (3) for the wavelength of 343 nm:

$$D = 0.012 \cdot C_{Cr} + 0.0091 \ (2)$$

$$D = 0.0081 \cdot C_{Cr} - 0.0022 \ (3)$$

wherein D is optical density, $\lg(I_0/I)$; $C_{Cr}$ is chromium concentration in the catalytic system solution (mol/l).

The apparent molar extinction coefficient $\varepsilon$ is equal to about 2100 l/mol Cr*cm (343 nm) based on total chromium contents.

**Example 3. Ethylene oligomerization using a step of measuring the catalytic system concentration**

The catalytic system was obtained according to technique described in Example 1. Then, the catalytic system was diluted with cyclohexane. In a pipeline at the feeding line for feeding diluted catalytic system to the ethylene trimerization reactor a flow cell for UV analysis was installed. A preliminarily diluted catalytic system solution was passed through this cell, and measurement of its optical density was carried out, followed by feeding of the diluted catalytic system to the ethylene trimerization reactor. The total mass of chromium fed to the reactor starting from certain time moment is determined according to the formula (4):

$$M(Cr, tN) = \sum_{i=0}^{N} \Delta t_i \cdot C_i \cdot V_i \ (4)$$

wherein M (Cr, tN) is chromium mass fed to the reactor to the N moment of time, $\Delta t_i$ is the i-th time period, $C_i$ is chromium mass concentration at the $t_i$ moment of time, $V_i$ is volumetric rate of feeding the diluted catalytic system.

Maintaining the chromium concentration at the constant level (2 ml/g) provides for a selectivity with respects to hexene-1 (to all isomers thereof) above 99.5%, and the productivity of the oligomerization process reaches 200 kg olefin per 1 g Cr per hour.

**Claims**

1. A method of online determination of chromium concentration in the olefin oligomerization catalytic system comprising a chromium source, a nitrogen-containing ligand and an alkyl aluminium, the method comprising the following steps:

    - diluting a catalytic system with hydrocarbon solvent to a required total chromium concentration ranging from 0.5 to 10 mg/l; and
    - determining chromium concentration in the diluted catalytic system by measuring optical density of the obtained diluted solution in the wavelength range of 400 to 300 nm at least 60 min after producing the catalytic system.

2. A method of claim 1 **characterized in that** the dilution is carried out to the concentration of catalytic system in the range of 2 to 5 mg/l.

3. A method of claim 1 or 2 **characterized in that** the catalytic system is diluted with a hydrocarbon solvent selected from aliphatic or cycloaliphatic hydrocarbons comprising 6 to 16 carbon atoms, in particular such as heptane, cyclohexane, decane, undecane, iso-decane fraction, hexene- 1, Isopar™ (ExxonMobil) or mixtures thereof.

4. A method of claim 1 wherein the catalytic system is obtained by mixing the chromium source and nitrogen-containing ligand followed by mixing thereof with alkyl aluminium, with the alkyl aluminium being additionally activated using UHF radiation (microwave radiation) prior to mixing with other components of the catalytic system.

5. A method of claim 4 **characterized in that** the frequency of the UHF radiation (microwave radiation) is selected in the range of 0.2 to 20 GHz, preferably is 2.45 GHz.

6. A method of claim 4 **characterized in that** the time period of the UHF irradiation (microwave irradiation) is from 20 seconds to 20 minutes.

7. A method of any one of claims 4 to 6 **characterized in that** mixing of the alkyl aluminium activated using UHF irradiation (microwave irradiation) with chromium source and nitrogen-containing ligand is carried out no more than 3 minutes after the irradiation stops, preferably, no more than 1 minute after the irradiation stops.

8. A method of claim 1 **characterized in that** the chromium source is a compound of general formula CrXn wherein each X may be identical or different organic substituent, preferably organic substituent comprising 1 to 20 carbon atoms independently selected from the group comprising alkyl group, alkoxy group, carboxy group, acetyl acetonate, amino group, amido group, or inorganic substituents selected from a group comprising chromium halides, chromium sulfates, chromium oxides and n is an integer from 1 to 6.

9. A method of claim 8 **characterized in that** the chromium source compound is a compound selected from the group comprising chromium (III) chloride, chromium (III) acetate, chromium (III) 2-ethylhexanoate, chromium (III) acetyl acetonate, chromium (III) pyrrolide, chromium (II) acetate, chromium (VI) dioxide dichloride ($CrO_2Cl_2$).

10. A method of claim 1 **characterized in that** the nitrogen-containing ligand is an organic compound comprising a pyrrole ring, preferably an organic compound selected from a group comprising pyrrole, 2,5-dimethylpyrrole, lithium pyrrolide (C4H4NLi), 2-ethylpyrrole, indole, 2-methylindole, 4,5,6,7-tetrahydroindole.

11. A method of claim 1 **characterized in that** the alkyl aluminium is a compound selected from a group comprising triethyl aluminium, diethyl aluminium chloride, tripropyl aluminium, triisobutyl aluminium, diethyl aluminium ethoxide, ethyl aluminium sesquichloride or mixtures thereof.

12. A method of any one of the claims 1 to 11 **characterized in that** the aluminium : chromium molar ratio in the catalytic system is 5:1 to 500:1, preferably 10:1 to 100:1, most preferably 20:1 to 50:1.

13. A method of any one of claims 1 to 12 **characterized in that** the ligand : chromium molar ratio in the catalytic system is from 2:1 to 50:1, preferably 2.5:1 to 5:1.

14. A method of claim 1 **characterized in that** the online determination of chromium concentration is carried out in closed type cells in the inert, air oxygen-free atmosphere.

15. A method of controlling chromium concentration in olefin oligomerization catalytic system comprising a chromium source, a nitrogen-containing ligand and alkyl aluminium, the method comprising:

- diluting a catalytic system comprising a chromium source, a nitrogen- containing ligand and alkyl aluminium with hydrocarbon solvent to a required concentration ranging from 0.5 to 10 mg/l;
- online determining chromium concentration in the diluted catalytic system by a method as claimed in any one of claims 1 to 14; and
- correcting, if necessary, the amount of solvent fed for dilution of the catalytic system and/or the amount of concentrated catalytic system in response to the performed measurement.

16. A process of olefin oligomerization comprising the following steps:

a) producing a catalytic system by mixing a chromium source, a nitrogen- containing ligand and an alkyl aluminium,
b) diluting the catalytic system obtained in step (a) with hydrocarbon solvent to a required concentration;
c) feeding diluted catalytic system, a feedstock comprising alpha-olefin and optionally a zinc compound to an oligomerization reactor;
d) reacting the feedstock comprising alpha-olefin under oligomerization conditions in the presence of a catalytic system comprising the chromium source, the nitrogen- containing ligand, alkyl aluminium and optionally zinc compounds,

**characterized in that** chromium concentration in a diluted catalytic system is controlled by the method of claim 15prior to feeding the diluted catalytic system to the oligomerization reactor (step c).

17. The process of claim 16**characterized in that** the diluted of the catalytic system is carried out to the required chromium concentration in the range of 0.5 to 10 mg/l, preferably 2 to 5 mg/l.

18. The process of claim 16 or 17 **characterized in that** the catalytic system is diluted with hydrocarbon solvent selected from aliphatic or cycloaliphatic hydrocarbons comprising 6 to 16 carbon atoms, in particular such as heptane, cyclohexane, decane, undecane, iso-decane fraction, hexene- 1, Isopar™ (ExxonMobil) or mixtures thereof.

19. The process of claim 16**characterized in that** the catalytic system is obtained by mixing the chromium source and nitrogen-containing ligand followed by mixing thereof with alkyl aluminium, with the alkyl aluminium being additionally activated using UHF radiation (microwave radiation) prior to mixing with other components of the catalytic system.

20. The process of claim 19 **characterized in that** the frequency of the UHF radiation (microwave radiation) is selected in the range of 0.2 to 20 GHz, preferably is 2.45 GHz.

21. The process of claim 19 **characterized in that** the time period of the UHF irradiation (microwave irradiation) is from 20 seconds to 20 minutes.

22. The process of any one of claims 16 to 19 **characterized in that** the mixing of the alkyl aluminium activated using UHF irradiation (microwave irradiation) with chromium source and nitrogen-containing ligand is carried out no more than 3 minutes after the irradiation stops, preferably, no more than 1 minute after the irradiation stops.

23. The process of claim 16 **characterized in that** the chromium source is a compound of general formula CrXn wherein each X may be identical or different organic substituent, preferably organic substituent comprising 1 to 20 carbon atoms independently selected from the group comprising alkyl group, alkoxy group, carboxyl group, acetyl acetonate, amino group, amido group, or inorganic substituents selected from a group comprising chromium halides, chromium sulfates, chromium oxides and n is an integer from 1 to 6.

24. The process of claim 23 **characterized in that** a compound selected from a group comprising chromium (III) chloride, chromium (III) acetate, chromium (III) 2-ethylhexanoate, chromium (III) acetyl acetonate, chromium (III) pyrrolide, chromium (II) acetate, chromium (VI) dioxide dichloride (CrO2Cl2) is used as the chromium source compound.

25. The process of claim 16 **characterized in that** the organic compound comprising a pyrrole ring is used as nitrogen-containing ligand, preferably an organic compound selected from a group comprising pyrrole, 2,5-dimethylpyrrole, lithium pyrrolide (C4H4NLi), 2-ethylpyrrole, indole, 2-methylindole, 4,5,6,7-tetrahydroindole is used as nitrogen- containing ligand.

26. The process of claim 16 **characterized in that** a compound selected from a group comprising triethyl aluminium, diethyl aluminium chloride, tripropyl aluminium, triisobutyl aluminium, diethyl aluminium ethoxide, ethyl aluminium sesquichloride or mixtures thereof is used as alkyl aluminium.

27. The process of claim 16 **characterized in that** that a compound selected from a group comprising metallic zinc, zinc-copper couple, activated zinc, alkyl zinc compounds, in particular such as dimethyl zinc, diethyl zinc, and dibutyl zinc, aryl zinc compounds, in particular such as diphenyl zinc and ditolyl zinc, zinc amides, in particular such as zinc pyrrolides and zinc-porphyrin complexes, zinc oxygenates, in particular such as formate, acetate, basic acetate and 2-ethylhexanoate, zinc halides, in particular such as anhydrous zinc chloride, or combinations thereof, is used as the zinc compound.

28. The process of any one of claims 16 to 27 **characterized in that** the aluminium : chromium molar ratio in the catalytic system is 5:1 to 500:1, preferably 10:1 to 100: 1, most preferably 20:1 to 50:1.

29. The process of any one of claims 16 to 28 **characterized in that** the ligand : chromium molar ratio in the catalytic system is 2:1 to 50:1, preferably 2.5:1 to 5:1.

30. The process of any one of claims 16 to 29 **characterized in that** the zinc : chromium molar ratio in the catalytic system is 2:1 to 100:1, preferably 5:1 to 50:1.

31. The process of any one of claims 16 to 30 **characterized in that** the olefin oligomerization process is carried out at a temperature in the range of 0°C inclusively to 160 °C inclusively, preferably 40°C inclusively to 130 °C inclusively, most preferably 80°C inclusively to 120 °C inclusively.

32. The process of any one of claims 16 to 31 **characterized in that** the olefin oligomerization process is carried out in the absence of water and oxygen.

33. The process of any one of claims 16 to 31 **characterized in that** the alpha-olefin is selected from ethylene (ethene), propylene (propene) and butylene (butene).

34. A device for controlling chromium concentration in a catalytic system comprising a chromium source, a nitrogen-containing ligand and alkyl aluminium comprising:

   - a catalytic system preparation unit connected downstream via a regulating flow meter with a mixing unit for mixing the catalytic system and a solvent;
   - a reservoir with a hydrocarbon solvent for diluting the catalytic system, connected downstream via a regulating flow meter with a mixing unit for mixing the catalytic system and the solvent;
   - an UV analyzer designed for measuring the optical density of the catalytic system diluted solution in a wavelength range of 400 to 300 nm after the diluted catalytic system exits the mixing unit; and
   - a feedback unit for carrying out correction of volumetric flow rates of the solvent fed from the solvent reservoir to the mixing unit and/or the catalyst solution fed from the catalytic system preparation unit to the mixing unit in response to measurement made by UV analyzer.

**35.** Use of the device of claim 34 in the olefin oligomerization method of claim 16.

**Patentansprüche**

**1.** Verfahren zur Online-Bestimmung der Chromkonzentration in einem katalytischen System zur Olefin-Oligomerisierung, das eine Chromquelle, einen stickstoffhaltigen Liganden und ein Alkylaluminium umfasst, wobei das Verfahren die folgenden Schritte umfasst:

- Verdünnen eines katalytischen Systems mit Kohlenwasserstofflösungsmittel auf eine erforderliche Gesamtchromkonzentration im Bereich von 0,5 bis 10 mg/1; und
- Bestimmung der Chromkonzentration in dem verdünnten katalytischen System durch Messung der optischen Dichte der erhaltenen verdünnten Lösung im Wellenlängenbereich von 400 bis 300 nm mindestens 60 min nach Herstellung des katalytischen Systems.

**2.** Verfahren nach Anspruch 1, **dadurch gekennzeichnet, dass** die Verdünnung auf eine Konzentration des katalytischen Systems im Bereich von 2 bis 5 mg/1 durchgeführt wird.

**3.** Verfahren nach Anspruch 1 oder 2, **dadurch gekennzeichnet, dass** das katalytische System mit einem Kohlenwasserstofflösungsmittel verdünnt wird, das aus aliphatischen oder cycloaliphatischen Kohlenwasserstoffen mit 6 bis 16 Kohlenstoffatomen ausgewählt ist, insbesondere wie Heptan, Cyclohexan, Decan, Undecan, Isodecan Fraktion, Hexen-1, Isopar™ (ExxonMobil) oder deren Mischungen.

**4.** Verfahren nach Anspruch 1, wobei das katalytische System durch Mischen der Chromquelle und des stickstoffhaltigen Liganden und anschließendes Mischen mit Alkylaluminium erhalten wird, wobei das Alkylaluminium vor dem Mischen mit anderen Komponenten des katalytischen Systems zusätzlich mit UHF-Strahlung (Mikrowellenstrahlung) aktiviert wird.

**5.** Verfahren nach Anspruch 4, **dadurch gekennzeichnet, dass** die Frequenz der UHF-Strahlung (Mikrowellenstrahlung) im Bereich von 0,2 bis 20 GHz, vorzugsweise bei 2,45 GHz, gewählt wird.

**6.** Verfahren nach Anspruch 4, **dadurch gekennzeichnet, dass** die Zeitdauer der UHF-Bestrahlung (Mikrowellenbestrahlung) 20 Sekunden bis 20 Minuten beträgt.

**7.** Verfahren nach einem der Ansprüche 4 bis 6, **dadurch gekennzeichnet, dass** das Mischen des durch UHF-Bestrahlung (Mikrowellenbestrahlung) aktivierten Alkylaluminiums mit der Chromquelle und dem stickstoffhaltigen Liganden nicht mehr als 3 Minuten nach Beendigung der Bestrahlung, vorzugsweise nicht mehr als 1 Minute nach Beendigung der Bestrahlung, durchgeführt wird.

**8.** Verfahren nach Anspruch 1, **dadurch gekennzeichnet, dass** die Chromquelle eine Verbindung der allgemeinen Formel CrXn ist, wobei jedes X ein identischer oder unterschiedlicher organischer Substituent sein kann, vorzugsweise ein organischer Substituent, der 1 bis 20 Kohlenstoffatome umfasst, die unabhängig voneinander aus der Gruppe ausgewählt sind, die eine Alkylgruppe, eine Alkoxygruppe, eine Carboxygruppe, Acetylacetonat, eine Aminogruppe, eine Amidogruppe oder anorganische Substituenten umfasst, die aus einer Gruppe ausgewählt sind, die Chromhalogenide, Chromsulfate und Chromoxide umfasst, und n eine ganze Zahl von 1 bis 6 ist.

**9.** Verfahren nach Anspruch 8, **dadurch gekennzeichnet, dass** die Chromquellenverbindung eine Verbindung ist, die aus der Gruppe ausgewählt ist, die Chrom(III)-chlorid, Chrom(III)-acetat, Chrom(III)-2-ethylhexanoat, Chrom(III)-acetylacetonat, Chrom(III)-pyrrolid, Chrom(II)-acetat, Chrom(VI)-dioxiddichlorid (CrO2Ch) umfasst.

**10.** Verfahren nach Anspruch 1, **dadurch gekennzeichnet, dass** der stickstoffhaltige Ligand eine organische Verbindung ist, die einen Pyrrolring umfasst, vorzugsweise eine organische Verbindung, die aus einer Gruppe ausgewählt ist, die Pyrrol, 2,5-Dimethylpyrrol, Lithiumpyrrolid (C4H4NLi), 2-Ethylpyrrol, Indol, 2-Methylindol, 4, 5, 6, 7-Tetrahydroindol umfasst.

**11.** Verfahren nach Anspruch 1, **dadurch gekennzeichnet, dass** das Alkylaluminium eine Verbindung ist, die aus einer Gruppe ausgewählt ist, die Triethylaluminium, Diethylaluminiumchlorid, Tripropylaluminium, Triisobutylaluminium, Diethylaluminiumethoxid, Ethylaluminiumsesquichlorid oder Mischungen davon umfasst.

**12.** Verfahren nach einem der Ansprüche 1 bis 11, **dadurch gekennzeichnet, dass** das molare Verhältnis von Aluminium zu Chrom in dem katalytischen System 5:1 bis 500:1, vorzugsweise 10:1 bis 100:1, am meisten bevorzugt 20:1 bis 50:1 beträgt.

**13.** Verfahren nach einem der Ansprüche 1 bis 12, **dadurch gekennzeichnet, dass** das molare Verhältnis von Ligand zu Chrom im katalytischen System 2:1 bis 50:1, vorzugsweise 2,5:1 bis 5:1 beträgt.

**14.** Verfahren nach Anspruch 1, **dadurch gekennzeichnet, dass** die Online-Bestimmung der Chromkonzentration in geschlossenen Zellen in einer inerten, luftsauerstofffreien Atmosphäre durchgeführt wird.

**15.** Verfahren zur Kontrolle der Chromkonzentration in einem katalytischen System zur Olefin-Oligomerisierung, das eine Chromquelle, einen stickstoffhaltigen Liganden und Alkylaluminium umfasst, wobei das Verfahren Folgendes umfasst:

- Verdünnen eines katalytischen Systems, das eine Chromquelle, einen stickstoffhaltigen Liganden und Alkylaluminium umfasst, mit einem Kohlenwasserstofflösungsmittel auf eine erforderliche Konzentration im Bereich von 0,5 bis 10 mg/1;
Online-Bestimmung der Chromkonzentration in dem verdünnten katalytischen System durch ein Verfahren nach einem der Ansprüche 1 bis 14; und
- gegebenenfalls Korrektur der Menge des zur Verdünnung des katalytischen Systems zugeführten Lösungsmittels und/oder der Menge des konzentrierten katalytischen Systems in Abhängigkeit von der durchgeführten Messung.

**16.** Prozess zur Olefin-Oligomerisierung, umfassend die folgenden Schritte:

a) Herstellung eines katalytischen Systems durch Mischen einer Chromquelle, eines stickstoffhaltigen Liganden und eines Alkylaluminiums,
b) Verdünnen des in Schritt (a) erhaltenen katalytischen Systems mit Kohlenwasserstofflösungsmittel auf eine erforderliche Konzentration;
c) Einspeisen des verdünnten katalytischen Systems, eines alpha-Olefin umfassenden Ausgangsmaterials und gegebenenfalls einer Zinkverbindung in einen Oligomerisierungsreaktor;
d) Umsetzen des alpha-Olefin umfassenden Einsatzmaterials unter Oligomerisierungsbedingungen in Gegenwart eines katalytischen Systems, das die Chromquelle, den stickstoffhaltigen Liganden, Alkylaluminium und gegebenenfalls Zinkverbindungen umfasst, **dadurch gekennzeichnet, dass** die Chromkonzentration in einem verdünnten katalytischen System durch das Verfahren nach Anspruch 15 vor dem Einspeisen des verdünnten katalytischen Systems in den Oligomerisierungsreaktor gesteuert wird

(Schritt c).

**17.** Prozess nach Anspruch 16, **dadurch gekennzeichnet, dass** die Verdünnung des katalytischen Systems auf die erforderliche Chromkonzentration im Bereich von 0,5 bis 10 mg/1, vorzugsweise 2 bis 5 mg/1, durchgeführt wird.

**18.** Prozess nach Anspruch 16 oder 17, **dadurch gekennzeichnet, dass** das katalytische System mit einem Kohlenwasserstofflösungsmittel verdünnt wird, das aus aliphatischen oder cycloaliphatischen Kohlenwasserstoffen mit 6 bis 16 Kohlenstoffatomen ausgewählt ist, wie z. B. Heptan, Cyclohexan, Decan, Undecan, Isodecan-Fraktion, Hexen-1, Isopar™ (ExxonMobil) oder Mischungen davon.

**19.** Prozess nach Anspruch 16, **dadurch gekennzeichnet, dass** das katalytische System durch Mischen der Chromquelle und des stickstoffhaltigen Liganden und anschließendes Mischen mit Alkylaluminium erhalten wird, wobei das Alkylaluminium vor dem Mischen mit anderen Komponenten des katalytischen Systems zusätzlich mit UHF-Strahlung (Mikrowellenstrahlung) aktiviert wird.

**20.** Prozess nach Anspruch 19, **dadurch gekennzeichnet, dass** die Frequenz der UHF-Strahlung (Mikrowellenstrahlung) im Bereich von 0,2 bis 20 GHz, vorzugsweise 2,45 GHz, gewählt wird.

**21.** Prozess nach Anspruch 19, **dadurch gekennzeichnet, dass** die Zeitdauer der UHF-Bestrahlung (Mikrowellenbestrahlung) 20 Sekunden bis 20 Minuten beträgt.

**22.** Prozess nach einem der Ansprüche 16 bis 19, **dadurch gekennzeichnet, dass** das Mischen des durch UHF-Bestrahlung (Mikrowellenbestrahlung) aktivierten Alkylaluminiums mit der Chromquelle und dem stickstoffhaltigen Liganden nicht länger als 3 Minuten nach Beendigung der Bestrahlung, vorzugsweise nicht länger als 1 Minute nach Beendigung der Bestrahlung, durchgeführt wird.

**23.** Prozess nach Anspruch 16, **dadurch gekennzeichnet, dass** die Chromquelle eine Verbindung der allgemeinen Formel CrXn ist, in der jedes X ein identischer oder unterschiedlicher organischer Substituent sein kann, vorzugsweise ein organischer Substituent, der 1 bis 20 Kohlenstoffatome umfasst, die unabhängig voneinander aus der Gruppe ausgewählt sind, die eine Alkylgruppe, eine Alkoxygruppe, eine Carboxylgruppe, ein Acetylacetonat, eine Aminogruppe, eine Amidogruppe oder anorganische Substituenten umfasst, die aus einer Gruppe ausgewählt sind, die Chromhalogenide, Chromsulfate und Chromoxide umfasst, und n eine ganze Zahl von 1 bis 6 ist.

**24.** Prozess nach Anspruch 23, **dadurch gekennzeichnet, dass** eine Verbindung, ausgewählt aus einer Gruppe, die Chrom(III)-chlorid, Chrom(III)-acetat, Chrom(III)-2-ethylhexanoat, Chrom(III)-acetylacetonat, Chrom(III)-pyrrolid, Chrom(II)-acetat, Chrom(VI)-dioxiddichlorid (CrO2Cl2) umfasst, als Chromquellenverbindung verwendet wird.

**25.** Prozess nach Anspruch 16, **dadurch gekennzeichnet, dass** die organische Verbindung, die einen Pyrrolring umfasst, als stickstoffhaltiger Ligand verwendet wird, vorzugsweise eine organische Verbindung, die aus einer Gruppe ausgewählt ist, die Pyrrol, 2,5-Dimethylpyrrol, Lithiumpyrrolid (C4H4NLi), 2-Ethylpyrrol, Indol, 2-Methylindol, 4,5,6,7-Tetrahydroindol umfasst, als stickstoffhaltiger Ligand verwendet wird.

**26.** Prozess nach Anspruch 16, **dadurch gekennzeichnet, dass** als Alkylaluminium eine Verbindung verwendet wird, die aus einer Gruppe ausgewählt ist, die Triethylaluminium, Diethylaluminiumchlorid, Tripropylaluminium, Triisobutylaluminium, Diethylaluminiumethoxid, Ethylaluminiumsesquichlorid oder Mischungen davon umfasst.

**27.** Prozess nach Anspruch 16, **dadurch gekennzeichnet, dass** eine Verbindung, ausgewählt aus einer Gruppe, die metallisches Zink, Zink-Kupfer-Paar, aktiviertes Zink, Alkylzinkverbindungen, insbesondere wie Dimethylzink, Diethylzink und Dibutylzink, Arylzinkverbindungen, insbesondere wie Diphenylzink und Ditolylzink, Zinkamide, insbesondere wie Zinkpyrrolide und Zinkporphyrinkomplexe, Zinkoxygenate, insbesondere wie Formiat, Acetat, basisches Acetat und 2-Ethylhexanoat, Zinkhalogenide, insbesondere wie wasserfreies Zinkchlorid, oder Kombinationen davon, als die Zinkverbindung verwendet wird.

**28.** Prozess nach einem der Ansprüche 16 bis 27, **dadurch gekennzeichnet, dass** das molare Verhältnis von Aluminium zu Chrom in dem katalytischen System 5:1 bis 500:1, vorzugsweise 10:1 bis 100:1, am meisten bevorzugt 20:1 bis 50:1 beträgt.

**29.** Prozess nach einem der Ansprüche 16 bis 28, **dadurch gekennzeichnet, dass** das molare Verhältnis von Ligand zu Chrom im katalytischen System 2:1 bis 50:1, vorzugsweise 2,5:1 bis 5:1 beträgt.

**30.** Prozess nach einem der Ansprüche 16 bis 29, **dadurch gekennzeichnet, dass** das molare Verhältnis von Zink zu Chrom im katalytischen System 2: 1 bis 100: 1, vorzugsweise 5: 1 bis 50: 1 beträgt.

**31.** Prozess nach einem der Ansprüche 16 bis 30, **dadurch gekennzeichnet, dass** das Olefin-Oligomerisierungsverfahren bei einer Temperatur im Bereich von 0°C einschließlich bis 160°C einschließlich, vorzugsweise 40°C einschließlich bis 130°C einschließlich, am meisten bevorzugt 80°C einschließlich bis 120°C einschließlich, durchgeführt wird.

**32.** Prozess nach einem der Ansprüche 16 bis 31, **dadurch gekennzeichnet, dass** das Olefin-Oligomerisierungsverfahren in Abwesenheit von Wasser und Sauerstoff durchgeführt wird.

**33.** Prozess nach einem der Ansprüche 16 bis 31, **dadurch gekennzeichnet, dass** das Alpha-Olefin ausgewählt ist aus Ethylen (Ethen), Propylen (Propen) und Butylen (Buten).

**34.** Prozess zur Kontrolle der Chromkonzentration in einem katalytischen System, das eine Chromquelle, einen stickstoffhaltigen Liganden und Alkylaluminium enthält, umfassend:

- eine Einheit zur Vorbereitung des katalytischen Systems, die stromabwärts über einen regulierenden Durchflussmesser mit einer Mischeinheit zum Mischen des katalytischen Systems und eines Lösungsmittels verbunden ist;
- ein Reservoir mit einem Kohlenwasserstofflösungsmittel zur Verdünnung des katalytischen Systems, das stromabwärts über einen regulierenden Durchflussmesser mit einer Mischeinheit zum Mischen des katalytischen Systems und des Lösungsmittels verbunden ist;
- einen UV-Analysator zur Messung der optischen Dichte der verdünnten Lösung des katalytischen Systems in einem Wellenlängenbereich von 400 bis 300 nm, nachdem das verdünnte katalytische System die Mischeinheit verlassen hat; und
- eine Rückkopplungseinheit zum Durchführen einer Korrektur der volumetrischen Durchflussraten des aus dem Lösungsmittelbehälter in die Mischeinheit zugeführten Lösungsmittels und/oder der aus der Katalysatorsystem-Vorbereitungseinheit in die Mischeinheit zugeführten Katalysatorlösung in Reaktion auf die vom UV-Analysator durchgeführte Messung.

**35.** Verwendung der Vorrichtung nach Anspruch 34 bei dem Olefin-Oligomerisierungsverfahren nach Anspruch 16.

**Revendications**

**1.** Méthode de détermination en ligne de la concentration de chrome dans le système catalytique d'oligomérisation des oléfines comprenant une source de chrome, un ligand contenant de l'azote et un alkylaluminium, la méthode comprenant les étapes suivantes:

- dilution d'un système catalytique avec un solvant hydrocarboné jusqu'à une concentration totale de chrome requise comprise entre 0,5 et 10 mg/1; et
- déterminer la concentration de chrome dans le système catalytique dilué en mesurant la densité optique de la solution diluée obtenue dans la gamme de longueurs d'onde de 400 à 300 nm au moins 60 minutes après la production du système catalytique.

2. Méthode de la revendication 1, **caractérisée par le fait que** la dilution est effectuée jusqu'à une concentration de système catalytique comprise entre 2 et 5 mg/1.

3. Méthode de la revendication 1 ou 2 **caractérisée en ce que** le système catalytique est dilué avec un solvant hydrocarboné choisi parmi les hydrocarbures aliphatiques ou cycloaliphatiques comprenant de 6 à 16 atomes de carbone, notamment tels que l'heptane, le cyclohexane, le décane, l'undécane, la fraction iso-décane, l'hexène- 1, l'Isopar™ (ExxonMobil) ou leurs mélanges.

4. Méthode de la revendication 1, dans laquelle le système catalytique est obtenu en mélangeant la source de chrome et le ligand contenant de l'azote, puis en les mélangeant avec de l'aluminium alkylique, l'aluminium alkylique étant en outre activé à l'aide d'un rayonnement UHF (rayonnement micro-ondes) avant d'être mélangé avec d'autres composants du système catalytique.

5. Méthode de la revendication 4 **caractérisée par le fait que** la fréquence du rayonnement UHF (rayonnement micro-ondes) est choisie dans la gamme de 0,2 à 20 GHz, de préférence à 2,45 GHz.

6. Méthode de la revendication 4 **caractérisée par le fait que** la durée de l'irradiation UHF (irradiation par micro-ondes) est comprise entre 20 secondes et 20 minutes.

7. Procédé de l'une quelconque des revendications 4 à 6, **caractérisé par le fait que** le mélange de l'alkylaluminium activé par irradiation UHF (irradiation par micro-ondes) avec la source de chrome et le ligand contenant de l'azote est effectué au maximum 3 minutes après l'arrêt de l'irradiation, de préférence au maximum 1 minute après l'arrêt de l'irradiation.

8. Méthode de la revendication 1 **caractérisée par le fait que** la source de chrome est un composé de formule générale CrXn dans laquelle chaque X peut être un substituant organique identique ou différent, de préférence un substituant organique comprenant 1 à 20 atomes de carbone choisis indépendamment dans le groupe comprenant un groupe alkyle, un groupe alcoxy, un groupe carboxy, un acétyl acétonate, un groupe amino, un groupe amido, ou des substituants inorganiques choisis dans un groupe comprenant des halogénures de chrome, des sulfates de chrome, des oxydes de chrome et n est un nombre entier de 1 à 6.

9. Méthode de la revendication 8 **caractérisée par le fait que** le composé source de chrome est un composé choisi dans le groupe comprenant le chlorure de chrome (III), l'acétate de chrome (III), le 2-éthylhexanoate de chrome (III), l'acétylacétonate de chrome (III), le pyrrolide de chrome (III), l'acétate de chrome (II), le dichlorure de dioxyde de chrome (VI) (CrO2Ch).

10. Méthode de la revendication 1 **caractérisée par le fait que** le ligand azoté est un composé organique comprenant un cycle pyrrole, de préférence un composé organique choisi dans un groupe comprenant le pyrrole, le 2,5-diméthylpyrrole, le pyrrolide de lithium (C4H4NLi), le 2-éthylpyrrole, l'indole, le 2-méthylindole, le 4, 5, 6, 7-tétrahydroindole.

11. Méthode de la revendication 1 **caractérisée par le fait que** l'alkyl aluminium est un composé choisi dans un groupe comprenant le triéthyl aluminium, le chlorure de diéthyl aluminium, le tripropyl aluminium, le triisobutyl aluminium, l'éthoxyde de diéthyl aluminium, le sesquichlorure d'éthyl aluminium ou des mélanges de ceux-ci.

12. Méthode de l'une quelconque des revendications 1 à 11, **caractérisée par le fait que** le rapport molaire aluminium/chrome dans le système catalytique est compris entre 5:1 et 500:1, de préférence entre 10:1 et 100:1, de préférence encore entre 20:1 et 50:1.

13. Méthode de l'une des revendications 1 à 12, **caractérisée par le fait que** le rapport molaire ligand/chrome dans le système catalytique est compris entre 2:1 et 50:1, de préférence entre 2,5:1 et 5:1.

14. Méthode de la revendication 1 **caractérisée par le fait que** la détermination en ligne de la concentration de chrome est effectuée dans des cellules de type fermé dans une atmosphère inerte, sans oxygène.

15. Méthode de contrôle de la concentration de chrome dans un système catalytique d'oligomérisation d'oléfines comprenant une source de chrome, un ligand contenant de l'azote et de l'aluminium alkylique, la méthode comprenant:

- diluer un système catalytique comprenant une source de chrome, un ligand contenant de l'azote et de l'alkylaluminium avec un solvant hydrocarboné jusqu'à une concentration requise comprise entre 0,5 et 10 mg/1;

- déterminer en ligne la concentration de chrome dans le système catalytique dilué par une méthode telle que revendiquée dans l'une quelconque des revendications 1 à 14; et

- corriger, si nécessaire, la quantité de solvant utilisée pour la dilution du système catalytique et/ou la quantité de système catalytique concentré en fonction de la mesure effectuée.

16. Procédé d'oligomérisation d'oléfines comprenant les étapes suivantes:

a) production d'un système catalytique par mélange d'une source de chrome, d'un ligand contenant de l'azote et d'un alkylaluminium,
b) dilution du système catalytique obtenu à l'étape a) avec un solvant hydrocarboné jusqu'à la concentration requise;
c) alimenter un réacteur d'oligomérisation avec le système catalytique dilué, une charge comprenant de l'alpha-oléfine et éventuellement un composé de zinc ;
d) faire réagir la charge comprenant l'alpha-oléfine dans des conditions d'oligomérisation en présence d'un système catalytique comprenant la source de chrome, le ligand contenant de l'azote, l'alkylaluminium et éventuellement des composés de zinc, **caractérisé par le fait que** la concentration de chrome dans un système catalytique dilué est contrôlée par la méthode de la revendication 15avant d'alimenter le réacteur d'oligomérisation avec le système catalytique dilué.

(étape c).

17. Procédé de la revendication 16 **caractérisé par le fait que** la dilution du système catalytique est effectuée jusqu'à la concentration de chrome requise dans la plage de 0,5 à 10 mg/1, de préférence de 2 à 5 mg/1.

18. Procédé de la revendication 16 ou 17 **caractérisé par le fait que** le système catalytique est dilué avec un solvant hydrocarboné choisi parmi les hydrocarbures aliphatiques ou cycloaliphatiques comprenant de 6 à 16 atomes de carbone, en parti9ulier tels que l'heptane, le cyclohexane, le décane, l'undécane, la fraction iso-décane, l'hexène- 1, l'Isopar™ (Exxon-Mobil) ou leurs mélanges.

19. Procédé de la revendication 16 **caractérisé par le fait que** le système catalytique est obtenu en mélangeant la source de chrome et le ligand contenant de l'azote, puis en les mélangeant avec de l'aluminium alkylique, l'aluminium alkylique étant en outre activé à l'aide d'un rayonnement UHF (rayonnement micro-ondes) avant d'être mélangé avec d'autres composants du système catalytique.

20. Procédé de la revendication 19 est **caractérisé par le fait que** la fréquence du rayonnement UHF (rayonnement micro-ondes) est choisie dans la gamme de 0,2 à 20 GHz, de préférence à 2,45 GHz.

21. Procédé de la revendication 19 **caractérisé par le fait que** la durée de l'irradiation UHF (irradiation par micro-ondes) est comprise entre 20 secondes et 20 minutes.

22. Procédé de l'une des revendications 16 à 19 **caractérisé par le fait que** le mélange de l'alkyl aluminium activé par irradiation UHF (irradiation par micro-ondes) avec la source de chrome et le ligand contenant de l'azote est effectué au maximum 3 minutes après l'arrêt de l'irradiation, de préférence au maximum 1 minute après l'arrêt de l'irradiation,

23. Procédé de la revendication 16 **caractérisé par le fait que** la source de chrome est un composé de formule générale CrXn dans lequel chaque X peut être un substituant organique identique ou différent, de préférence un substituant organique comprenant 1 à 20 atomes de carbone choisis indépendamment dans le groupe comprenant un groupe alkyle, un groupe alkoxy, un groupe carboxyle, un acétyl acétonate, un groupe amino, un groupe amido, ou des substituants inorganiques choisis dans un groupe comprenant les halogénures de chrome, les sulfates de chrome, les oxydes de chrome et n est un nombre entier de 1 à 6.

24. Procédé de la revendication 23 **caractérisé en ce qu'**un composé choisi dans un groupe comprenant le chlorure de chrome (III), l'acétate de chrome (III), le 2-éthylhexanoate de chrome (III), l'acétylacétonate de chrome (III), le pyrrolide de chrome (III), l'acétate de chrome (II), le dichlorure de dioxyde de chrome (VI) (CrO2Cl2) est utilisé en tant que composé source de chrome,

25. Procédé de la revendication 16 **caractérisé par le fait que** le composé organique comprenant un cycle pyrrole est utilisé comme ligand contenant de l'azote, de préférence un composé organique choisi dans un groupe comprenant le pyrrole, le 2,5-diméthylpyrrole, le pyrrolide de lithium (C4H4NLi), le 2-éthylpyrrole, l'indole, le 2-méthylindole, le 4,5,6,7-tétrahydroindole, est utilisé comme ligand contenant de l'azote.

26. Procédé de la revendication 16 **caractérisé par le fait qu'**un composé choisi dans un groupe comprenant l'aluminium triéthyle, le chlorure d'aluminium diéthyle, l'aluminium tripropyle, l'aluminium triisobutyle, l'éthoxyde d'aluminium diéthyle, le sesquichlorure d'aluminium éthyle ou leurs mélanges est utilisé en tant qu'aluminium alkylique.

27. Procédé de la revendication 16, **caractérisé par le fait qu'**un composé choisi dans un groupe comprenant le zinc métallique, le couple zinc-cuivre, le zinc activé, les composés d'alkyl-zinc, . en particulier tels que le diméthyl zinc, le diéthyl zinc et le dibutyl zi'nc, les composés aryl zinc, en particulier tels que le diphényl zinc et le ditolyl zinc, les amides de zinc, en particulier tels que les pyrrolides de zinc et les complexes zinc-porphyrine, les oxygénés de zinc, en particulier tels que le formiate, l'acétate, l'acétate basique et le 2-éthylhexanoate, les halogénures de zinc, en particulier tels que le chlorure de zinc anhydre, ou des combinaisons de ceux-ci, est utilisé comme composé de zinc.

28. Le procédé de la revendication 16 à 27 est **caractérisé par le fait que** le rapport molaire aluminium/chrome dans le système catalytique est compris entre 5:1 et 500:1, de préférence entre 10:1 et 100:1, de préférence encore entre 20:1 et 50:1.

29. Procédé de la revendication 16 à 28 **caractérisé par le fait que** le rapport molaire ligand/chrome dans le système catalytique est compris entre 2:1 et 50:1, de préférence entre 2,5:1 et 5:1.

30. Procédé de la revendication 16 à 29 **caractérisé par le fait que** le rapport molaire zinc/chrome dans le système catalytique est compris entre 2:1 et 100:1, de préférence entre 5:1 et 50:1.

31. Procédé de la revendication 16 à 30 **caractérisé par le fait que** le procédé d'oligomérisation des oléfines est effectué à une température comprise entre 0°C inclus et 160°C inclus, de préférence entre 40°C inclus et 130°C inclus, de préférence encore entre 80°C inclus et 120°C inclus.

32. Procédé de la revendication 16 à 31 **caractérisé par le fait que** le processus d'oligomérisation des oléfines est effectué en l'absence d'eau et d'oxygène.

33. Procédé de la revendication 16 à 31 **caractérisé par le fait que** l'alpha-oléfine est choisie parmi l'éthylène (éthène), le propylène (propène) et le butylène (butène).

34. Dispositif de contrôle de la concentration de chrome dans un système catalytique comprenant une source de chrome, un ligand contenant de l'azote et de l'alkylaluminium, comprenant:

- une unité de préparation du système catalytique reliée en aval par un débitmètre régulateur à une unité de mélange du système catalytique et d'un solvant;
- un réservoir contenant un solvant hydrocarboné pour diluer le système catalytique, relié en aval par un débitmètre régulateur à une unité de mélange pour mélanger le système catalytique et le solvant;
- un analyseur UV conçu pour mesurer la densité optique de la solution diluée du système catalytique dans une gamme de longueurs d'onde de 400 à 300 nm après que le système catalytique dilué a quitté l'unité de mélange; et
- une unité de rétroaction pour corriger les débits volumétriques du solvant envoyé du réservoir de solvant à l'unité de mélange et/ou de la solution catalytique envoyée de l'unité de préparation du système catalytique à l'unité de mélange en réponse aux mesures effectuées par l'analyseur UV.

35. Utilisation du dispositif de la revendication 34 dans la méthode d'oligomérisation des oléfines de la revendication 16.

FIG.1

**FIG.2**

EP 3 717 890 B1

Catalytic system formation kinetics

FIG.3

EP 3 717 890 B1

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016105227 A
- US 7315369 B
- US 7396970 B
- US 7505129 B
- US 9040605 B
- RU 2104088

### Non-patent literature cited in the description

- **IGOR Y. SKOBELEV ; VALENTINA N. PANCHENKO ; OLEG Y. LYAKIN ; KONSTANTIN P. BRYLIAKOV ; VLADIMIR A. ZAKHAROV ; EVGENII P. TALSI.** In situ EPR monitoring of chromium species formed during Cr-pyrrolyl ethylene trimerization catalyst formation. *Organometallics,* 2010, vol. 29 (13), 2943-2950

- **MARKUS KREYE ; CONSTANTIN G. DANILIUC ; MATTHIAS FREYTAG ; PETER G. JONES ; MARC D. WALTER.** Coordination chemistry of sterically encumbered pyrrolyl ligands to chromium (II): mono(pyrrolyl)-chromium and diazachromocene formation. *Dalton Trans,* 2014, vol. 43, 9052-9060